# EUROPÄISCHE PATENTANMELDUNG

(11) **EP 2 471 532 A2**
(43) Veröffentlichungstag der Anmeldung: **04.07.2012**
(21) Anmeldenummer: 11193075.6
(22) Anmeldetag: 12.12.2011
(51) Int. Cl.: A61K 31/403, A61K 41/00, A61N 5/067

(54) **Wirkstoff, der Indocyaningrün und/oder Infracyaningrün enthält**

(30) Priorität: 03.01.2011 DE 102011000020
(71) Anmelder: A.R.C. Laser GmbH, 90411 Nürnberg (DE)
(72) Erfinder: Thyzel, Reinhardt, 90542 Eckental / Eckenhaid (DE)
(74) Vertreter: Schröer, Gernot H.

(57) **Zusammenfassung**

Wirkstoff, der Indocyaningrün und/oder Infracyaningrün enthält, zur Verwendung in der Behandlung dentaler oder paradontologischer Infektionserkrankungen unter Bestrahlung mit Licht, insbesondere Laserlicht, aus einem Wellenlängenbereich zwischen 790 nm und 830 nm.

## Beschreibung

Die Erfindung betrifft einen Wirkstoff, der Indocyaningrün und/oder Infracyaningrün enthält.

Indocyaningrün (ICG von engl. indocyanine green) ist gemäß www.wikipedia.de ein fluoreszierender Farbstoff, der in der Medizin als Indikatorsubstanz bei Herz-, Kreislauf-, Leber- und Augenerkrankungen eingesetzt wird. Dabei wird es intravenös verabreicht und in Abhängigkeit von der Leberleistung mit einer Halbwertszeit von ca. 3 - 4 Minuten aus dem Körper eliminiert. ICG-Natriumsalz liegt normalerweise in Pulverform vor und kann in unterschiedlichen Lösungsmitteln gelöst werden; meist wird zur besseren Löslichkeit 5 % (<5 % je nach Charge) Natriumiodid beigegeben.

Für Jodunverträglichkeiten ist auch ein iodidfreies Indocyaningrün bekannt, das als Infracyaningrün (IfCG oder IFC green) bezeichnet wird.

Ferner gibt es gemäß www.wikipedia.de erste Untersuchungen in der Dermatologie, die darauf hindeuten, dass ICG unter bestimmten Umständen - wie passender Laserstärke und bestimmter Wellenlängen - die Eigenschaft innehat, eine photodynamische Reaktion auszulösen und somit mittelfristig auch als PDT-Therapeutikum eingesetzt werden könnte. Hierzu gibt es erste Untersuchungen zur dermatologischen Anwendung von Christoph Abels, Sonja Fickweiler, Petra Weiderer, Wolfgang Bäumler, Ferdinand Hofstädter, Michael Landthaler and R.-M. Szeimies: Indocyanine green (ICG) and laser irradiation induce photooxidation. In: Archives of Dermatological Research. Springer Berlin / Heidelberg, Vol. 292, Number 8, August 2000, sowie in R.-M. Szeimies, T. Lorenzen, S. Karrer, C. Abels und A. Plettenberg: Photochemotherapie kutaner Aids-assoziierter Kaposi-Sarkome mit Indocyaningrün und Laserlicht. In: Der Hautarzt, Springer, Berlin / Heidelberg, Vol. 52, Number 4, März 2001, S. 322-326 Das Absorptions- und Fluoreszenzspektrum von ICG liegt im nahinfraroten Bereich. Beide sind in starkem Maße von dem verwendeten Lösungsmittel und der Konzentration abhängig. ICG absorbiert hauptsächlich zwischen 600 nm und 900 nm und emittiert Fluoreszenz zwischen 750 nm und 950 nm. Die große Überlappung der Absorptions- und Fluoreszenzspektren führt zu einer starken Reabsorption der Fluoreszenz durch ICG selbst.

Das Fluoreszenzspektrum ist recht breit. Seine Maxima liegen in Wasser bei ca. 810 nm und in Blut bei ca. 830 nm. Für medizinische Anwendungen, die auf Absorption basieren, ist das Absorptionsmaximum bei ca. 800 nm (in Blutplasma bei kleinen Konzentrationen) wichtig. In Kombination mit Fluoreszenzdetektion wird häufig auch mit Lasern der Wellenlänge um 780 nm gearbeitet. Bei dieser Wellenlänge absorbiert ICG noch sehr gut, und es ist trotzdem noch technisch möglich, das Anregungslicht zu unterdrücken, um die Fluoreszenz zu detektieren.

In der Zahnheilkunde besteht ein Problem bei der Behandlung von Zahnfeleisch und Zahnfleischtaschen darin, dass Bakterien in die Blutbahn gelangen können und dadurch zu schwerwiegenden, mitunter tödlichen Infektionen führen können. deshalb werden hier begeleitend Antibiotika eingesetzt, die oral verabreicht werden.

In einem Firmenprospekt "Die antimikrobielle Photodynamische Therapie (PDT)" von Dr. Mettraux und Prof. Dr. Dr. Dörtbudak von einer Fa ms dental unter dem Handelsnamen Helbo^{®} eine antimikrobielle photodynamische Therapie zur Behandlung von Paradontitis marginalis, Periimplantitis und Karies sowie zur endodontischen Therapie angeboten mit dem Photosensitizer-Wirkstoff Helbo blue^{®}, der auf Methylenblau beruht, in Kombination mit einem Laser mit 670 nm Wellenlänge und 75 mW Leistung sowie mit einer Lichtleitfaser, deren Faserspitze mittels einer die Spitze umgebenden Mikrolinse das rote Licht radiär mit gleichmäßiger Lichtverteilung abstrahlt. Das zu behandelnde Zahnfleisch wird mit dem Wirkstoff Helbo blue^{®} benetzt und dann mit dem darüber in den Mund gehaltenen Lichtleiterende mit dem Laserlicht bestrahlt, wodurch bakterielle Keime abgetötet werden.

Die vorliegende Erfindung beruht nun auf der aus Versuchen gewonnenen Erkenntnis, dass ICG zur Behandlung von Infektionen am Zahnfleisch und an Zähnen unter Bestrahlung mit Licht, insbesondere Laserlicht, mit einer Wellenlänge im Bereich von 780 bis 830 nm eine hervorragende antimikrobiotische, insbesondere antimikrobielle, Wirkung entfaltet.

Gemäß Patentanspruch 1 wird deshalb ein Wirkstoff, der Indocyaningrün (ICG) und/oder Infracyaningrün (IfCG) enthält, zur Verwendung in der Behandlung dentaler oder paradontologischer Infektionserkrankungen unter Bestrahlung mit Licht, insbesondere Laserlicht, aus einem Wellenlängenbereich zwischen 790 nm und 830 nm, insbesondere zwischen 800 nm und 820 nm, vorgeschlagen. Mit anderen Worten, der Wirkstoff wird zur photodynamischen Therapie am Zahnfleisch im Munde eines Patienten eingesetzt, um dort Bakterien und andere Mikroorganismen zu bekämpfen, wobei die Wirkung des ICG oder IfCG gegen die Bakterien, Pilze oder andere Mikroorganismen durch die Bestrahlung mit Licht, insbesondere Laserlicht, aus dem angegebenen Wellenlängenspektrum induziert wird. Es wird also erfindungsgemäß eine weitere medizinische Indikation für den Wirkstoff ICG und eine erste medizinische Indikation für den Wirkstoff IfCG beansprucht.

Ausgestaltungen und Weiterbildungen der Erfindung ergeben sich aus den abhängigen Patentansprüchen.

Der Wirkstoff enthält bevorzugt wenigstens ein Lösungsmittel, in der Regel Wasser, insbesondere anionenarmes Wasser, zur Lösung des ICG oder IfCG, wobei die Konzentration des ICG und/oder IfCG im Lösungsmittel wenigstens 250 µg bezogen auf 1 ml Lösungsmittel beträgt. Diese vergleichsweise hohe Konzentration ist vorteilhaft, um Verdünnungen des Wirkstoffes im Mundraum aufgrund des Mundspeichels auszugleichen. Vorzugsweise wird die Konzentration des ICG oder IfCG noch höher gewählt und beträgt dann wenigstens 500 µg, jedoch in der Regel maximal 5000 µg, bezogen auf 1 ml Lösungsmittel.

Spezielle und vorteilhafte Verwendungen des Wirkstoffes sind bei der Wunddesinfektion bei Zahnextraktionen oder bei der endodontischen Behandlung oder Behandlung von Infektionen in Zahnwurzelkanälen oder bei der Behandlung von Periimplantitis vorgesehen.

Besonders vorteilhaft ist die Anwendung des Wirkstoffes in der Behandlung von Infektionen am Zahnfleisch oder zur Bekämpfung von Bakterien und/oder Pilzen am Zahnfleisch, insbesondere beim Reinigen von Zahntaschen oder ans Zahnfleisch angrenzender Zwischenräume zwischen den Zähnen und Zahnfleisch

Das Licht, insbesondere Laserlicht, weist bei der Verwendung des Wirkstoffes eine Dauerleistung oder Bestrahlungsstärke größer als 100 mW, vorzugsweise aus einem Bereich von 100 mW bis 200 mW und/oder für wenigstens 30 s, auf und/oder einen Energieeintrag oder eine Energiedichte größer 30 J/cm², vorzugsweise zwischen 30 J/cm² und 60 J/cm².

In einer besonders vorteilhaften Weiterbildung wird für die Verwendung des Wirkstoffes ein System zur Behandlung dentaler oder paradontologischer Infektionserkrankungen vorgeschlagen, das wenigstens einen Wirkstoffapplikator zur Applikation des Wirkstoffes gemäß der Erfindung und wenigstens einen Lichtapplikator zum Bestrahlen des applizierten Wirkstoffes mit Licht, insbesondere Laserlicht, aus einem Wellenlängenbereich zwischen 790 nm und 830 nm, insbesondere 800 nm bis 820 nm.

Das System umfasst bevorzugt eine Lichtquelle, insbesondere einer Laserlichtquelle, die bei der Behandlung vorzugsweise eine Dauerleistung oder Bestrahlungsstärke größer als 100 mW, vorzugsweise aus einem Bereich von 100 mW bis 200 mW und/oder für wenigstens 30 s, erzeugt und/oder einen Energieeintrag oder eine Energiedichte größer 30 J/cm², vorzugsweise zwischen 30 J/cm² und 60 J/cm² liefert.

Insbesondere dient das System auch zur Ablösung oder Abtrennung oder Nekrotisierung von Entzündungszahnfleischgewebe, in dem sich der Wirkstoff befindet.

## Patentansprüche

1. Wirkstoff, der Indocyaningrün und/oder Infracyaningrün enthält, zur Verwendung in der Behandlung dentaler oder paradontologischer Infektionserkrankungen unter Bestrahlung mit Licht, insbesondere Laserlicht, aus einem Wellenlängenbereich zwischen 790 nm und 830 nm.

2. Wirkstoff nach Anspruch 1 mit wenigstens einem Lösungsmittel, insbesondere Wasser, insbesondere anionenarmes Wasser, wobei die Konzentration des Indocyaningrün und/oder Infracyaningrün im Lösungsmittel wenigstens 250 µg, vorzugsweise wenigstens 500 µg, auf 1 ml Lösungsmittel beträgt, insbesondere maximal 5000 µg auf 1 ml Lösungsmittel beträgt.

3. Wirkstoff nach einem der vorhergehenden Ansprüche zur Verwendung in der Wunddesinfektion bei Zahnextraktion.

4. Wirkstoff nach einem der vorhergehenden Ansprüche zur Verwendung in der endodontischen Behandlung oder der Behandlung von Infektionen in Zahnwurzelkanälen.

5. Wirkstoff nach einem der vorhergehenden Ansprüche zur Verwendung in der Behandlung von Periimplantitis.

6. Wirkstoff nach einem der vorhergehenden Ansprüche zur Verwendung in der Behandlung von Infektionen am Zahnfleisch oder zur Bekämpfung von Bakterien und/oder Pilzen am Zahnfleisch, insbesondere beim Reinigen von Zahntaschen oder ans Zahnfleisch angrenzender Zwischenräume zwischen den Zähnen und Zahnfleisch.

7. Wirkstoff nach einem der vorhergehenden Ansprüche, wobei das zur Bestrahlung vorgesehene Licht, insbesondere Laserlicht, eine Dauerleistung oder Bestrahlungsstärke größer als 100 mW, vorzugsweise aus einem Bereich von 100 mW bis 200 mW und/oder für wenigstens 30 s, auf und/oder einen Energieeintrag oder eine Energiedichte größer 30 J/cm² vorzugsweise zwischen 30 J/cm² und 60 J/cm² aufweist.

8. System zur Behandlung dentaler oder paradontologischer Infektionserkrankungen mit einem wenigstens einem Wirkstoffapplikator zur Applikation des Wirkstoffes nach einem der vorhergehenden Ansprüche und mit wenigstens einem Lichtapplikator zum Bestrahlen des applizierten Wirkstoffes mit Licht, insbesondere Laserlicht, aus einem Wellenlängenbereich zwischen 790 nm und 830 nm.

9. System nach Anspruch 8 mit einer Lichtquelle, insbesondere einer Laserlichtquelle, die bei der Behandlung eine Dauerleistung oder Bestrahlungsstärke größer als 100 mW, vorzugsweise aus einem Bereich von 100 mW bis 200 mW und/oder für wenigstens 30 s, erzeugt und/oder einen Energieeintrag oder eine Energiedichte größer 30 J/cm², vorzugsweise zwischen 30 J/cm² und 60 J/cm² liefert, wobei der Lichtapplikator an die Lichtquelle angekoppelt oder ankoppelbar ist. Das System umfasst bevorzugt eine Lichtquelle, insbesondere einer Laserlichtquelle, die bei der Behandlung vorzugsweise eine.

10. System nach einem der Ansprüche 7 bis 9 zur Ablösung oder Abtrennung oder Nekrotisierung von Entzündungszahnfleischgewebe, in dem sich der Wirkstoff befindet.
